Europäisches Patentamt

European Patent Office

Office européen des brevets

(19)

(11) Publication number: **0 494 929 B1**

## EUROPEAN PATENT SPECIFICATION

(45) Date of publication of patent specification: **23.08.95**

(51) Int. Cl.6: **C12N 15/15**, C12P 21/02, A61K 38/57, C12N 15/62

(21) Application number: **90914972.6**

(22) Date of filing: **03.10.90**

(86) International application number:
**PCT/NL90/00145**

(87) International publication number:
**WO 91/05048 (18.04.91 91/09)**

(54) **MUTANTS OF THE HUMAN PLASMINOGEN ACTIVATOR INHIBITOR 1 (PAI-1), THEIR PREPARATION AND USE.**

(30) Priority: **03.10.89 NL 8902454**

(43) Date of publication of application:
**22.07.92 Bulletin 92/30**

(45) Publication of the grant of the patent:
**23.08.95 Bulletin 95/34**

(84) Designated Contracting States:
**AT BE CH DE DK ES FR GB IT LI LU NL SE**

(56) References cited:
**EP-A- 0 190 652**
**EP-A- 0 260 757**
**EP-A- 0 326 013**

**J. Cell. Biochem., Volume 0, Suppl. 12B, 30 January-26 February 1988, H.E. SHUBEITA et al.: "Molecular Studies on Plasminogen Activator Inhibitor by Site Directed Mutagenesis", see page 293, Abstract X 317**

(73) Proprietor: **Stichting Centraal Laboratorium van de Bloedtransfusiedienst van het Nederlandse Rode Kruis**
**Plesmanlaan 125**
**NL-1066 CX Amsterdam (NL)**

(72) Inventor: **PANNEKOEK, Hans**
**Uiterweg 126**
**NL-1431 AR Aalsmeer (NL)**

(74) Representative: **Smulders, Theodorus A.H.J., Ir. et al**
**Vereenigde Octrooibureaux**
**Nieuwe Parklaan 97**
**NL-2587 BN 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

J. Cell. Biochem., Volume 0, Suppl. 13E, 3-24 April 1989, R.D. GERARD et al.: "Structure and Function of the Plasminogen Activator Inhibitor-1 Reactive Center", see page 200, Abstract P 307

The Journal of Biological Chemistry, Volume 265, No. 22, 5 August 1990, The American Society for Biochemistry and Molecular Biology, Inc., (US), H.J. EHRLICH et al.: "Alteration of Serpin Specificity by a Protein Cofactor", pages 13029-13035 see the whole article, especially page 13031, table 1

**Description**

The invention relates to the fields of genetic engineering using the recombinant DNA technique, designing and preparing modified enzymes, and using these modified enzymes in medicine.

More particularly, the invention relates to mutants of the human plasminogen activator inhibitor 1 (PAI-1) with altered specificity towards serine proteases, and to a method of producing such mutants using genetically engineered organisms, in particular micro-organisms, and the use of the mutants in a fibrinolytic/thrombolytic therapy with a plasminogen activator.

It has appeared that a fibrinolytic therapy with a plasminogen activator, in particular the human tissue-type plasminogen activator t-PA, or the urokinase-type plasminogen activator u-PA, or non-natural constructs with plasminogen activator action is an effective tool for the treatment of thrombotic occlusion (clotting) of blood vessels, such as thrombosis of deep veins or a myocardial infarct. However, a serious problem in fibrinolytic/thrombolytic therapy is that soon after an initially successful thrombolysis, clotting occurs again (re-occlusion). In various studies re-occlusions were observed in 10-33% of cases after t-PA therapy for thrombosis in the coronary artery (Collen et al., 1984; Verstraete et al., 1988; TIMI study group, 1985; Verstraete et al., 1987)

Apparently activation of the coagulation system during fibrinolytic therapy leads to the formation of the enzyme thrombin, which subsequently induces re-occlusion on account of the fact that it promotes the formation of the insoluble protein fibrin from fibrinogen (Owen et al., 1988) and activates the blood platelets, which promotes the aggregation of blood platelets and the binding of blood platelets to fibrin (Kerins et al., 1988). The fact that reocclusion occurs even when the anticoagulent agent heparin is administered shows there is a strong need for therapeutic alternatives to this antigoagulent agent.

The plasminogen activator inhibitor type 1 (PAI-1) is considered to be the physiological inhibitor of both tissue-type plasminogen activator (t-PA) and urokinase-type plasminogen activator (u-PA). The PAI-1 is produced by cultured vascular endothelial cells and is present in the alpha-granuleS of blood platelets from which it is subsequently released upon activation (see Schleef and Loskutoff, 1988). A cloning of the entire PAI-1 cDNA and determining its nucleotide sequence have enabled the prediction of the amino acid sequence of the pre-PAI-1 protein, consisting of 402 amino acids (Pannekoek et al., 1986). It is an important fact that mature PAI-1 does not contain any cystine residues, which implies the possibility of a direct expression of biologically functional PAI-1 in the bacterium Escherichia coli without a reduction-oxydation procedure for disulphide bridges (Pannekoek et al., 1986). Further, a comparison between the amino acid sequence of PAI-1 and that of other proteins revealed that PAI-1 belongs to the protein super family of the serine protease inhibitors (the so-called serpins) (Pannekoek et al., 1986). It is supposed that the members of this inhibitor family descend from a common ancestor and, in view of the striking homology of their amino acid sequences, possess a similar three-dimensional structure (Carrell and Boswell, 1986). Serpins form an equimolar inactive complex with their respective target proteases, which complexes are sodium-dodecyl sulphate (SDS) resistant. The principle of serpin reactivity is based on a specific carboxy-terminal region of the molecule, namely the reactive site P1-P1' (for PAI-1, arginine in position 346 and methionine in position 347, respectively: see Andreasen et al., 1986), which is presented as a pseudo-substrate to the target serine protease (Travis and Salvesen, 1983). The specificity of a serpin towards different target proteases is caused at least in part by the sequence of and around the reactive centre, which strongly varies between the different serpins and has probably undergone much more rapid evolutionary variation than the remainder of the molecule (Hill and Hastie, 1987). The interaction between the serine protease and the serpin is accomplished by the serine residue in the active centre of the protease and the P1 residue of the serpin. The formation of a complex between the serine protease and the serpin ultimately leads to a hydrolysis of the peptide bond between the P1- and P1'residues. In contrast with this mechanism of cleavage of the real substrate by the protease, the serpin-protease complex is supposed to be a relatively stable intermediate in the proteolytic process, whose hydrolysis of the peptide bond between P1 and P1' proceeds extremely slowly (Travis and Salvesen, 1983).

Compared with the other serpins, PAI-1 secreted by the cultured vascular endothelial cells has a unique feature in that it does not only have an active conformation and an inactive cleaved conformation, but also is found mostly in a latent form (Hekman en Loskutoff, 1985). By a treatment with denaturants (Hekman and Loskutoff, 1985) or negatively-charged phospholipids (Lamoers et al., 1987) this latent form can be reactivated. Another property of PAI-1 is that in its active form it is deposited in the subendothelial matrix, where it is found in the form of a complex with the binding protein vitronectin (Mimuro and Loskutoff, 1989). Recently, binary complexes of PAI-1 with vitronectin have also been found in the plasma medium (Wiman et al., 1988; Declerck et al., 1988). It is known that association of these molecules in the subendothelial matrix or in the plasma leads to a partial stabilisation of the active form of PAI-1 (Mimuro et al., 1987;

Declerck et al., 1988). Furthermore, vitronectin and PAI-1 are both secreted by activated platelets, and subsequently found in the form of complexes. This observation indicates that vitronectin may function as a locally expressed regulator of the protease inhibition.

As part of a further study into determinants of protease specificity of serpins, particularly PAI-1, mutants have now been constructed from full-length PAI-1 cDNA, containing substitutions in the region of the reactive centre. These mutants were expressed in Escherichia coli bacteria, purified to homogeneity and activated. This study provided clear indications that specific mutations in and around the reactive sites effect the reactivity of PAI-1 towards its target serine protease t-PA and towards the serine protease thrombin, which normally is not a target of PAI-1. It appeared that compared with the wild-type enzyme (i.e. natural PAI-1) the mutants in question exhibit a greater reactivity towards thrombin, especially in the presence of vitronectin. From the results obtained it seems to follow that vitronectin is a protein cofactor for the activity of the serpin, strongly affecting the target specificity of the serpin PAI-1. The surprising finding that the PAI-1 mutants to be defined hereinafter become more or less effective thrombin inhibitors in the presence of vitronectin and at the same time exhibit a much smaller reactivity towards t-PA creates a real possibility of solving the problem of fibrinolytic/thrombolytic therapy as referred to hereinbefore. The physiological significance of the combination of properties of the new mutants according to the invention can be explained as follows. Modulation of thrombin activity occurs at the endothelial cell surface and involves thrombomodulin (Esmon, 1989) or heparin in conjunction with antithrombin III (Rosenberg and Damus, 1973). So far, regulation of thrombin activity at a growing platelet-rich thrombus has not been described. Recently, it has been demonstrated that upon activation of platelets with thrombin both vitronectin and PAI-1 are released and can be isolated as complexes. In such an environment the complexes can function as a relevant regulator of thrombin activity. This means that PAI-1 besides its known anti-fibrinolytic function, acting as the primary inhibitor of both t-PA and u-PA, also exhibits an unknown anti-coagulent activity. In wild-type PAI-1 this anti-coagulent activity is relatively weak, but the mutants described here have, in the presence of their co-factor vitronectin, the ability to effect to a greater or lesser extent an efficient inhibition of thrombin.

Since plasma contains a great amount of vitronectin operating as co-factor (the concentration of vitronectin in plasma is approximately 200 µg/ml) this property can be used in a thrombolytic therapy. When a PAI-1 mutant according to the invention is administered intravenously, it will bind to the vitronectin and thus become a strong thrombin inhibitor, while at the same time it will function much less effectively as an inhibitor of the plasminogen activator, which is the typical target of PAI-1. An additional advantage of the mutants according to the invention is that because PAI-1 binds to fibrin (Murayama and Bang, 1987; Keijer et al., 1988), the mutants will probably be directed to the thrombus (which is essentially composed of fibrin and platelets) in the form of their complex with vitronectin and there the mutants will subsequently inhibit the action of thrombin and thus prevent reocclusion.

Accordingly the invention primarily provides particular mutants of the human plasminogen activator inhibitor 1 (PAI-1), namely mutants in which the region of the reactive centre, which essentially consists of the amino acid sequence (according to the one-letter code) SGTVASSSTAVIVSARMAPEEIIMD, is replaced in whole or in part by the corresponding part of antithrombin III (ATIII), whose region of the reactive centre essentially consists of the amino acid sequence (according to the one-letter code) EGSEAAASTAVVIAGRSLNPNRVTFKAN.

Preferable are such mutants in which at least the amino acids RM in the positions P1 and P1' of PAI-1 are replaced by the amino acids RS of ATIII in corresponding positions, while more preferable are such mutants in which at least the amino acids ARMA in the positions P2 through P2' of PAI-1 are replaced by the amino acids GRSL of ATIII in corresponding positions. Most preferable are those mutants in which at least the amino acids SARMAP in the positions P3 through P3' of PAI-1 are replaced by the amino acids AGRSLN of ATIII in corresponding positions.

Although this preference might give the impression that the invention relates only to mutants in which a symmetrical substitution relative to the reactive centre P1-P1' has taken place, this is by no means the case and accordingly the invention also comprises more or less asymmetrical mutants.

The sites of the reactive centre mentioned above are specified in Table 1 both for ATIII and for PAI-1. The changes according to the invention are restricted to the region of the reactive centre, i.e. the region in Table 1 between the two boxes (EVNE and RPFL).

TABLE 1

Sequences of the region of the reactive centre of ATIII and PAI-1, aligned at maximum homology.

```
                                   P1P1'
ATIII   EVNE  EGSEAAASTAVVIAGR SLNPNRVTFKAN  RPFL
PAI-1   EVNE  SGTVASSSTAVIVSAR MAPEE---IIMD   RPFL
```

The invention does not only relate to the mutants proper, but includes recombinant polynucleotides, at least a part of which codes for the new PAI-1 mutants according to the invention. The recombinant polynucleotides according to the invention may consist of the mutated gene itself, or be composed of a vector part and an insertion part, the insertion part comprising a nucleotide sequence which codes for a PAI-1 mutant according to the invention. The term "recombinant polynucleotides" is intended to include both recombinant DNA and recombinant RNA. With the current state of the art mostly recombinant DNA will be involved, for instance in the form of a recombinant plasmid which is based on a vector which is suitable for cloning and/or expression in specific host organisms. For many kinds of host organisms suitable vectors are known, such as the various pUC plasmids for Escherichia coli bacteria. Host organisms transformed by means of such a recombinant polynucleotide can subsequently be used in a method for producing the PAI-1 mutant. This method for producing a PAI-1 mutant also falls within the scope of the invention.

In view of the above-mentioned physiological significance of the surprising properties of the mutants according to the invention, the invention further comprises pharmaceutical preparations which comprise a PAI-1 mutant according to the invention in combination with one or more pharmaceutically acceptable carriers and/or adjuvants, and the use of the PAI-1 mutants according to the invention in a fibrinolytic/thrombolytic therapy with a plasminogen activator for preventing the occurrence of reocclusion.

The invention will now be further explained with reference to the following experimental section and the corresponding Figures.

Description of the Figures

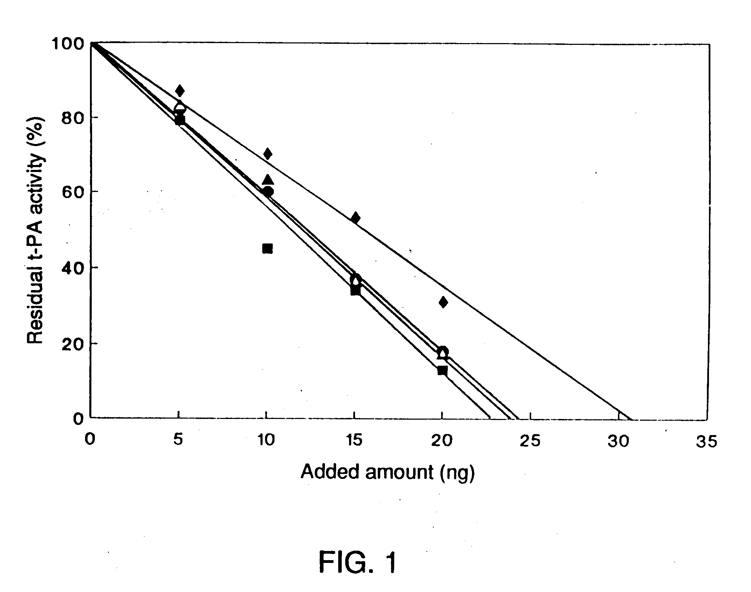

Fig. 1 shows the results of a titration of the t-PA inhibiting action of PAI-1 and mutants thereof. Different amounts of the various inhibitors were incubated for 1 h at 37°C with two-chain t-PA (1.5 nM) in a total volume of 50μl. Then 0.5 mM of the synthetic substrate H-D-ile-pro-arg-p-nitroanilide (S2288) was added and the residual tPA activity was determined from a linear plot in which the increase of the absorption at 405 nm was plotted against the incubation time. Fig. 1 shows how for the various inhibitors examined the residual t-PA activity (in %) varies with the amount of inhibitor added (in nanograms). The results for PAI-1 obtained from conditioned medium of cultured endothelial cells (ECCM-PAI-1) are represented by the squares, those for recombinant wild-type PAI-1 by rounds, those for the mutant PAI-1 P3-P3'ATIII by triangles and those for the mutant PAI-1 P1-P1'ATIII by diamonds.

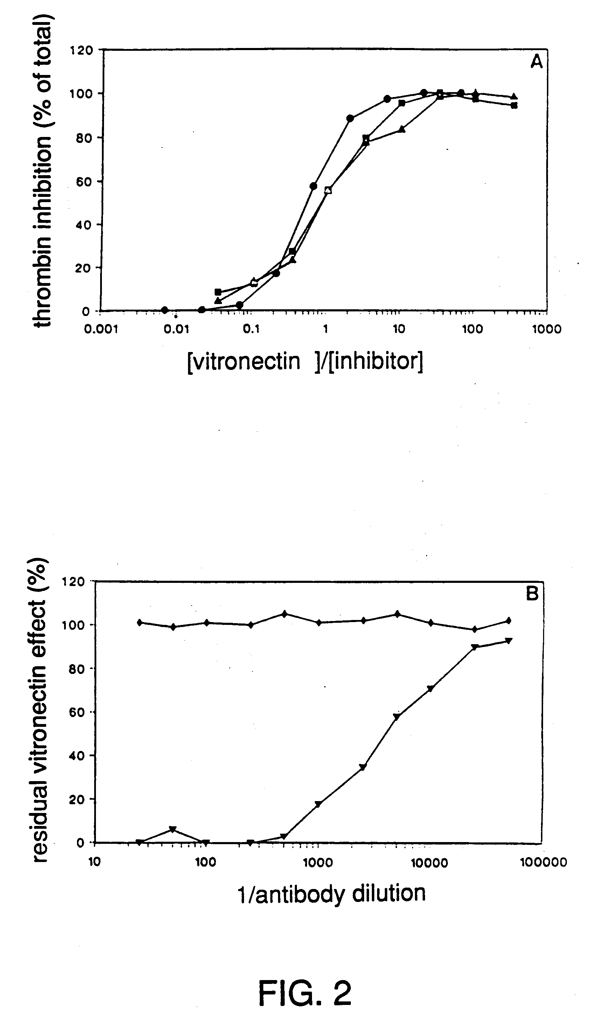

Fig. 2 shows the effect of vitronectin on the inhibition of thrombin both by wild-type PAI-1 and by the mutants according to the invention. To that end the mole ratio of vitronectin to (mutant) PAI-1 is plotted against the percentage of thrombin inhibition in graph A. The rounds indicate the results when 3.75 nM wild-type PAI-1 was incubated for 60 min with 0.15 nM thrombin and with the specified amounts of vitronectin. The triangles show the results for a similar experiment with 0.75 nM of mutant PAI-1 P1-P1'ATIII, while the squares indicate the results when 0.75 nM of mutant PAI-1 P3-P3'ATIII was incubated for 10 min with the above-mentioned concentrations of thrombin and vitronectin.

Graph B of Fig. 2 concerns experiments in which 0.15 nM thrombin was incubated for 1 h at 37°C with 0.75 nM of mutant PAI-1 P3-P3'ATIII in the presence or in the absence of 0.8 nM vitronectin. The residual thrombin activity was determined in the manner to be described hereinafter and fixed at approximately 50% in the presence of vitronectin and at 100% in the absence of vitronectin. A rabbit polyclonal antiserum (4 mg/ml) was raised against purified vitronectin (Preissner et al., 1985) and in part purified by ammonium sulphate precipitation and chromatography on DEAE-Sephacel. As a control were used rabbit immunoglobulins, raised against purified human Von Willebrand factor (Dakopatts, Glostrup, Denmark). Antisera were added to the reaction mixtures in the dilutions specified before vitronectin was added, and the residual thrombin activity was measured.

The residual thrombin activity in the absence of antisera but in the presence of 0.8 nM vitronectin was arbitrarily fixed at 100%. The triangles indicate the results in the case where anti-vitronectin was present, while the diamonds represent the results when anti-Von Willebrand was present.

Experimental section

Expression and purification of PAI-1 and PAI-1 mutants from Escherichia coli.

Two substitution mutants were constructed in which amino acid residues of and around the reactive site P1-P1' of PAI-1 were substituted by those of the thrombin inhibitor antithrombin III (ATIII); see Bock et al., 1982; Carrell and Boswell, 1986. The first mutant contained the P1-P1' sequence of antithrombin III, while the remainder of the PAI-1 protein was left unaltered. The second mutant contained the P3-P3' sequence of antithrombin III, while again the remainder of the PAI-1 protein was left unaltered. For the expression in transformed E. coli cells of PAI-1 and PAI-1 mutants the strictly regulated tryptophan promoter-operator was used, which leads to a high yield of 10-40 mg per liter cultured cells. After lysis of the cells PAI-1 and the mutants derived therefrom were purified using a three-step purification procedure in which Q-Sepharose Fast Flow chromatography and immunoaffinity chromatography with an immobilized anti-PAI-1 monoclonal antibody were carried out. Analysis of the proteins by electrophoresis on SDS-polyacrylamide gels (10% w/v polyacrylamide gel in which 0.2% w/v SDS) under non-reducing conditions and silver staining showed single protein bands (the results are not shown here). As expected, due to the absence of glycosylation in Escherichia coli the apparent molecular weight of the recombinant proteins was slightly lower than that of glycosylized PAI-1 from conditioned medium of cultured endothelial cells (ECCM-PAI-1; see Lambers et al., 1987). In an immunoblot analysis in which a murine monoclonal antibody was used, directed against purified human PAI-1, isolated from conditioned medium of cultured vascular endothelial cells, the same pattern was observed as in the protein staining (these results are not shown here either).

The purified inhibitors were used to determine the second-order rate constants with the target serine protease t-PA and the serine protease thrombin, which normally is not a target. The preparations were also used to examine the influence of vitronectin, the PAI-1 binding protein from the endothelial cel matrix and plasma, on the kinetic constants and the inhibition mechanism.

Inhibition of t-PA

The inhibitory ability of purified PAI-1, PAI-1 mutants and ECCM-PAI-1 for t-PA was determined by incubating the enzyme during a relatively long period of 1 h with an increasing amount of the various inhibitors, followed by a determination of the residual t-PA activity. As shown in Fig. 1, the inhibitory ability was virtually the same for all four inhibitors tested, which points to a comparable response to a guanidine-HCl induced activation. The second-order association rate constants for purified ECCM-PAI-1, PAI-1 and PAI-1 P1-P1'ATIII were virtually the same (3.4, 3.2 and 2.5 x $10^7$ $M^{-1}sec^{-1}$, respectively). This observation indicates that glycosylation of PAI-1 is not essential for the interaction with t-PA. Further, it appears the substitution of the methionine residue in the P1' position by serine, in view of the properties of the mutant PAI-1 P1-P1'ATIII, does not essentially effect the inhibition of t-PA. The PAI-1 P3-P3'ATIII inhibitor, on the other hand, has a 25-50 fold lower second-order rate constant (0.84 x $10^6$ $M^{-1}$ $sec^{-1}$) than PAI-1 for t-PA inhibition, which underscores the importance of the residues adjacent to P1-P1' for the specificity towards t-PA. It further appeared that the presence of a molar excess of vitronectin over PAI-1 does not in any of the inhibitors effect the second-order rate constant of the inhibitors with t-PA, which is in agreement with previously reported observations for ECCM-PAI-1 (Declerck et al., 1988).

Inhibition of thrombin

Before the second-order rate constants of PAI-1 and PAI-1/antithrombin III hybrids, i.e. PAI-1 P1-P1'ATIII and PAI-1 P3-P3'ATIII, with thrombin were determined, it was investigated whether the binding protein of PAI-1 influenced the interaction between PAI-1 or PAI-1 mutants and thrombin. Surprisingly, vitronectin appeared to significantly increase the thrombin inhibiting ability of PAI-1 and did so even much more strongly in the mutants, in particular the mutant PAI-1 P3-P3'ATIII. At an approximately equimolar concentration of vitronectin and PAI-1 a half-maximum inhibition of thrombin was observed, as shown in graph A of Fig. 2. When instead of vitronectin bovine serum albumin was used, no increase in the thrombin inhibition was observed (the results are not shown) and also in the presence of both vitronectin and an affinity purified polyclonal antibody, raised against purified vitronectin, no increase in the thrombin inhibition could be observed (see graph B in Fig. 2). The effect of vitronectin on the inhibition of thrombin by PAI-1 and mutants thereof is therefore very distinctly different from the effect on the inhibition on t-PA. In the latter case vitronectin leads only to a stabilization of the active form of PAI-1 by lengthening its half-value period 2-3 fold, without its inhibitory capacity being increased.

Then the second-order rate constants for PAI-1, PAI-1 P1-P1'ATIII and PAI-1 P3-P3'ATIII with thrombin in the presence or absence of an optimum concentration of vitronectin were determined. The results are shown in Table 2. It clearly shows that PAI-1 P3-P3'ATIII is a 10-fold better or faster inhibitor of thrombin than PAI-1, which indicates that the P3-P3' residues of antithrombin III are crucial to thrombin inhibition. By contrast, apparently, the requirements for the P1' residue, i.e. methionine in the case of PAI-1 or serine in the case of PAI-1 P1-P1'ATIII, are less important in thrombin inhibition, in a similar way as in t-PA inhibition. The rate of association with thrombin of both PAI-1 and the mutants derived thereof increases 100-200 fold in the presence of vitronectin, which results in comparable $k_1$ values for PAI-1, PAI-1 P1-P1'ATIII and ECCM-PAI-1 of 1.6-2.9 x $10^5$ $M^{-1}$ $sec^{-1}$ and for PAI-1 P3-P3'ATIII of 1.8 x $10^6$ $M^{-1}$ $sec^{-1}$. Again these results point to an apparently identical behaviour of PAI-1 of $\underline{E}$ $\underline{coli}$ origin and PAI-1 coming from endothelial cells.

Especially in the presence of vitronectin the mutant protein PAI-1 P3-P3'ATIII appears to function as an efficient thrombin inhibitor. The protein vitronectin seems to operate as a co-factor for inhibition of thrombin by PAI-1.

## Complex formation with thrombin

To determine whether the effect of vitronectin on thrombin inhibition is due to a dose dependent enhancement of the formation of SDS-stable complexes between protease and serpin, a $^{125}$I-labeled thrombin was incubated with PAI-1 or with the efficient thrombin inhibitor PAI-1 P3-P3'ATIII and increasing concentrations of vitronectin. The mixtures were then analyzed by electrophoresis on SDS-polyacrylamide gels under non-reducing conditions and subsequent fluorography (the results are not shown here). Increasing concentrations of vitronectin resulted in an increased formation of SDS-stable complexes between thrombin and each of the two serpins. More than 90% of thrombin was found in the complex under conditions of 3-fold excess of PAI-1 P3-P3'ATIII over thrombin and vitronectin at optimal concentration. Activation of PAI-1, for instance with guanidine-HCl, is a prerequisite for complex formation, because when the activation was omitted not any complexed thrombin was found (the data are not shown here). Consequently, the increase in the rate of association between thrombin and PAI-1 and mutants derived therefrom by vitronectin seems to be due to a more efficient formation of SDS-stable complexes between thrombin and the inhibitor.

The fact that under the experimental conditions employed the mutant PAI-1 P3-P3'ATIII is a threefold better inhibitor of thrombin than of t-PA renders it plausible that further mutants of the human plasminogen activator inhibitor 1 in which a larger part of the region of the reactive centre of PAI-1 is substituted by the corresponding part of ATIII are at least as promising adjuvants in a fibrinolytic/thrombolytic therapy.

Table 2

| Second-order rate constants (k1) for the inhibition of thrombin by PAI-1 and mutants thereof in the absence or presence of vitronectin | | |
|---|---|---|
| DERIVATIVE | VITRONECTIN | k1 ($M^{-1}.sec^{-1}$) |
| PAI-1 | - | 1.1 +/- 0.2 x $10^3$ |
| PAI-1 | + | 2.2 +/- 0.4 x $10^5$ |
| PAI-1 P1-P1'AT3 | - | 3.1 +/- 1.0 x $10^3$ |
| PAI-1 P1-P1'AT3 | + | 2.9 +/- 0.6 x $10^5$ |
| PAI-1 P3-P3'AT3 | - | 1.3 +/- 0.5 x $10^4$ |
| PAI-1 P3-P3'AT3 | + | 1.8 +/- 0.4 x $10^6$ |

The second-order rate constants (k1) were determined according to the procedure to be described hereinafter.

## Materials

Restriction endonucleases were purchased from New England Biolabs or Bethesda Research Laboratories. T4 polynucleotide kinase was obtained from Pharmacia (Uppsala, Sweden) and the Klenow fragment of DNA polymerase I from Boehringer Mannheim Biochemicals. The replicative form (RF) of M13mp18am4 DNA was obtained from Anglian Biotechnology Limited, Colchester, U.K. Sequenase DNA polymerase was purchased from United States Biochemicals. Casamino acids (lacking L-tryptophan) were obtained from Difco. The synthetic substrates H-D-isoleucineproline-arginine-p-nitroanilide (S2288) and H-D-phenylalaninepipecolyl-arginine-p-nitroanilide (S2238) were obtained from KabiVitrum (Stockholm, Sweden) and Q-Sepharose Fast Flow from Pharmacia (Uppsala, Sweden). Ampicillin and pancreatic DNAse I were from Sigma and Co., and goat anti-mouse IgG conjugated to alkaline phosphatase was from Promega. The plasmid pMBL11, containing the promotor-operator of the Escherichia coli tryptophan operon was obtained from the Medical Biological Laboratory TNO, Rijswijk, The Netherlands and Escherichia coli K12 strain 1046 (recA⁻) was obtained from the Phabagen Collection of the State University of Utrecht, The Netherlands. The murine monoclonal antibody MAI-13, directed against human PAI-1, covalently coupled to Sepharose was purchased from Biopool (Umea, Sweden).

## Proteins

Bowes melanoma-derived two-chain t-PA (91000 IU/mg) was purchased from Biopool (Umea, Sweden). Human thrombin, purified to apparent homogeneity, was obtained from the Central Laboratory of the Blood Transfusion Institute of the Netherlands Red Cross, Department of Blood Coagulation. Active site titration of purified thrombin with p-nitrophenyl p'-guanidino benzoate (Chase and Shaw, 1970) yielded a concentration of 5.0 mg/ml: this value agrees well with a determination of the protein concentration of 4.5 mg/ml, according to the Bradford procedure. Thrombin was labelled with a $^{125}$I label, using the Iodogen method, resulting in a specific radioactivity of 1,6 $\mu$Ci/$\mu$g protein. Vitronectin was purified from human plasma to apparent homogeneity as described previously by Preissner et al. in 1985. PAI-1 was purified from conditioned media of cultured, human umbilical vein endothelial cells (ECCM-PAI-1) using the three-step procedure described below for the purification of recombinant PAI-1 from lysates of cultured, transformed Escherichia coli cells.

## General methods

Standard molecular-biological techniques, including plasmid DNA and bacteriophage M13 DNA isolations, restriction fragment isolations, enzyme reactions with nucleic acids and bacterial transformations were performed as described by Maniatis et al. (1982). Nucleotide sequence determinations were performed using the dideoxy chain-termination method of Sanger et al., 1977, with Sequenase DNA polymerase. Synthetic oligonucleotides were synthesized by solid-phase phosphor amidite chemistry on an automated synthesizer (Applied Biosystems, model 381A)

## Construction of expression plasmids

Plasmids, which encode human PAI-1 protein and mutants thereof and which are suitable for expression of biologically functional PAI-1 proteins in transformed Escherichia coli, were constructed as follows. Full-length human PAI-1 cDNA was obtained from plasmid pUC8/PAIex, described previously by the inventors (Pannekoek et al., 1986). The entire nucleotide sequence of full-length PAI-1 cDNA and the derived PAI-1 amino acid sequence have previously been reported (Pannekoek et al., 1986). Plasmid pUC8/PAIex DNA was incubated with the restriction endonuclease ApaL1 and the generated fragments were made blunt-end, using the Klenow fragment of DNA polymerase I. Subsequently, the resulting DNA preparation was incubated with the restriction endonuclease BglII and the generated fragments were separated by agarose gel electrophoresis. The desired ApaL1 (blunt-ended)-BglII fragment (approximately 1300 base pairs), containing the entire PAI-1 coding sequence, was identified, isolated and purified from the gel, using standard procedures (Maniatis et al., 1982). Plasmid pMBL11 was employed as an expression vector for the various recombinant proteins in Escherichia coli. The choice of the expression vector is not particularly important for the findings described here. Many other, well-established expression vectors for Escherichia coli may perform equally well as the vector employed here. The vector pMBL11 carries the Escherichia coli tryptophan promotor-operator regulatory elements and the gene for beta-lactamase, providing for resistance against the antibiotic ampicillin. A multiple cloning site (5'ACTCGAGATCTAGGATCC 3'), containing unique restriction sites for the restriction endonucleases XhoI, BglII and BamHI was introduced immediately after the sixth codon of the trpE gene, i.e. the most promotor-proximal gene of the tryptophan operon. pMBL11 was cleaved with the restriction endonuclease BglII, subsequently made blunt-end with the Klenow fragment of DNA polymerase I and then treated with the restriction endonuclease BamHI. The resulting BglII (blunt-end)-BamHI fragment of approximately 4800 base pairs was isolated from an agarose gel after electrophoresis and ligated to the approximately 1300 base pair long ApaL1 (blunt-end)-BglII fragment, mentioned above, to yield plasmid pMBL11/PAI-1. The nucleotide sequence of the relevant, fused restriction sites was established by the method of DNA sequencing, outlined above.

Oligonucleotide-directed mutagenesis

Both strands of a linker molecule with the sequence (of the "upper" strand): 5' GAATTCGAATGCCGGCCCACCTGGCCATGCATCCATGGGTCGAC 3', containing the restriction sites EcoRI-BsmI-SfiI-NsiI-NcoI-SalI, were synthesized and employed to substitute for the EcoRI-SalI portion of the multiple cloning site of phage M13mp18am4 RF DNA. A (330 base pair ) SalI-NsiI restriction fragment from full-length, human PAI-1 cDNA (corresponding to positions 1045 and 1375, respectively) was inserted into this modified M13 mp18am4 RF. Site-directed mutagenesis on the resulting single-stranded template was carried out according to Kramer et al., 1984. To construct cDNA coding for a derivative of PAI-1in which P1' (methionine) of PAI-1 is replaced by the corresponding residue (serine) of antithrombin III (Bock et al., 1982; Carrell and Boswell, 1986), the following synthetic oligonucleotide was synthesized: 5' GTCATAGTCTCAGCCCGCTCAGCCCCCGAGGAGATCATC 3' to ultimately yield a mutant cDNA, denoted PAI-1 P1-P1'ATIII. To construct cDNA coding for a derivative of PAI-1 in which P3, P2, P1', P2' and P3' of PAI-1 are replaced by the amino acid residues of the corresponding sequence of antithrombin III, the following synthetic oligonucleotide was synthesized: 5' TCCACAGCTGTCATAGTCGCCGGAAGATCACTGAACGAGGAGATCATCATGGAC 3' to ultimately yield a mutant cDNA, denoted PAI-1 P3-P3'ATIII. The nucleotide sequences of the entire mutated SalI-NsiI restriction fragments were verified by DNA sequencing. Finally, the mutated NsiI-SalI fragments were isolated to substitute the non-mutated corresponding fragment of the expression plasmid pMBL11/PAI-1, to yield plasmids pPAI-1/P1-P1'-ATIII and pPAI-1/P3-P3'ATIII, respectively. The amino acid sequence around the reactive centre of PAI-1, and the amino acid substitutions of PAI-1 P1-P1'ATIII and PAI-1 P3-P3'ATIII, as compared to "wild-type" PAI-1, are presented in Table 3.

Table 3

| Amino acid sequences around the reactive centre of PAI-1, PAI-1 P1-P1' ATIII and PAI-1 P3-P3'ATIII | | | | | | | | |
|---|---|---|---|---|---|---|---|---|
| DERIVATIVE | P4 | P3 | P2 | P1 | P1' | P2' | P3' | P4' |
| | 343 | 344 | 345 | 346 | 347 | 348 | 349 | 350 |
| PAI-1 | val | ser | ala | arg | met | ala | pro | glu |
| PAI-1 P1-P1'ATIII | val | ser | ala | arg | ser | ala | pro | glu |
| PAI-1 P3-P3'ATIII | val | ala | gly | arg | ser | leu | asn | glu |

The establishment of the residues which constitute the reactive centre (P1-P1') has previously been described (Andreasen et al., 1986). In Table 3, amino acid residues are presented in the three-letter code. The difference between the amino acid sequence of wild-type PAI-1 and the mutant protein PAI-1 P1-P1'ATIII is the substitution of met-347 by ser-347. The mutant protein PAI-1 P3-P3'ATIII contains different amino acids compared with PAI-1 at positions P3, P2, P1', P2' and P3', whereas the remainder of the molecule is identical to wild type PAI-1.

Expression of PAI-1 and PAI-1 mutants in Escherichia coli

Escherichia coli K12 strain 1046, containing one of the plasmids described above, was grown overnight in LB (Maniatis et al., 1982) containing 100$\mu$g/ml ampicillin and 250 $\mu$g/ml L-tryptophan. The overnight culture was diluted 100-fold in the same medium, and cultured until a density of about 6 x$10^8$ cells/ml was

reached. Cells were collected by centrifugation, washed once with M9 medium (Maniatis et al., 1982) and finally resuspended in M9 medium supplemented with 0.5% glucose, 1 $\mu$g/ml thiamine, 100 $\mu$g/ml ampicillin and 0.2% casamino acids. Subsequently, expression based on derepression of the tryptophan operator was allowed to proceed for 16 to 20 h at 37°C and terminated by centrifugation of the cells.

Purification of PAI-1 and PAI-1 mutants from E.coli lysates

Bacterial pellets from a culture of 25 ml were suspended in 2.5 ml of a buffer containing 10 mM Tris-HCl, pH 8.0, 1 mM EDTA, 100 mM NaCl and 0.1% (v/v) Tween-80 and sonicated three times for 30 sec at 0°C. The lysates were centrifuged at 30.000 x g for 10 min, and, after addition of 1/100 volume of 1 M MgCl$_2$ incubated with 10 $\mu$g/ml pancreatic DNAse I for 30 min at 37°C. Then ammonium sulfate was added up to a final concentration of 25% (w/v) and the resulting pellet was discarded. The pellet of a subsequent ammonium sulfate addition till 45% (w/v) was suspended in 2.5 ml of 20 mM Tris-HCl, pH 8.0, 0.1% (v/v) Tween-80 and dialyzed overnight against the same buffer. The solution, containing PAI-1 or a derivative thereof, was adsorbed batchwise for 15 min at room temperature onto 2 ml Q-Sepharose Fast Flow resin, which was then packed into a column, washed with 10 ml 20 mM Tris-HCl (pH 8.0), 0.1% (v/v) Tween-80 and the inhibitors were eluted with 5 ml 20 mM TrisHCl (pH 8.0), 0.1% (v/v) Tween-80, 200 mM NaCl. For the final purification step the eluate was incubated batchwise for 12 h at 4°C with 1 ml suspension of the anti-PAI-1 monoclonal antibody MAI-13 covalently linked to Sepharose. Again, the resin was packed into a column, washed with 10 column volumes of 20 mM Tris-HCl (pH 8.0), 0.1% (v/v) Tween-80, 1 M NaCl and eluted with 3 ml 100 mM glycine (pH 3.3), 0.1% (v/v) Tween-80 and collected directly into 300 $\mu$l 1 M Tris-HCl (pH 8.0). The final homogeneous preparations of PAI-1 or mutants thereof were predominantly in the latent conformation.

Activation of PAI-1, PAI-1 mutants and titration of their respective activity

PAI-1 or PAI-1 mutants (20-30 $\mu$g/ml) were activated by incubation for 2 h at room temperature with 4 M guanidine-HCl (Hekman and Loskutoff, 1985). The samples were then dialyzed overnight against 20 mM Tris-HCl (pH 8.0), 100 mM NaCl, 0.1% (v/v) Tween-80. After dialysis, the concentration was adjusted to 10 $\mu$g/ml and arginine was added up to a final concentration of 50 mM to stabilize the active conformation. Increasing amounts of activated PAI-1 or PAI-1 mutants were incubated for 1 h at 37°C with 1.5 nM of two-chain t-PA. Then, the chromogenic substrate S2288 (0.5 mM) was added and residual t-PA activity was determined from a linear plot of increase of absorbence at 405 nm over time.

Determination of the second-order rate constants of PAI-1 and PAI-1 mutants with t-PA or thrombin

The second-order rate constants (k1) for the inhibition of t-PA with various inhibitors was determined as follows. 1.5 nM of two-chain t-PA (15 nM in experiments with PAI-1 P3-P3'ATIII) was incubated at 37°C in 20 mM Tris-HCl (pH 8.0), 100 mM NaCl, 0.1% (v/v) Tween-80 with 2 nM of ECCM-PAI-1 (derived from conditioned medium of endothelial cells), PAI-1, PAI-1 P1-P1'ATIII or 20 nM active PAI-1 P3-P3'ATIII. At designated times (5 sec to 16 min), the reaction was stopped by a 40-fold dilution of the reaction mixture with 1.2 mM S2288. Residual t-PA activity was determined from the initial increase of absorbence at 405 nm measured in a Cary 219 spectrophotometer. The second-order rate constant (k1) was calculated using a standard equation for a second-order reaction, under conditions of slight excess of inhibitor over enzyme:

$$k1.t = \frac{1}{Io-Eo} \left\{ \ln \left(1 + \frac{Io-Eo}{Et}\right) - \ln \frac{Io}{Eo} \right\}$$

wherein Eo and Io are the initial concentrations of enzyme and inhibitor, respectively, and Et is the enzyme concentration at time t.

The second-order rate constants for the inhibition of thrombin by PAI-1 or PAI-1 mutants were determined similarly as described above for inhibition of t-PA. For that purpose 2.5 nM of thrombin and 10 to 40 nM of the active component of one of the inhibitors were used. At designated times, the reaction was stopped by a 30-fold dilution with 0.6 mM of the chromogenic substrate S2238. In some experiments 66 nM vitronectin was added immediately prior to the addition of 10 nM PAI-1 or a mutant thereof.

## Complex formation of PAI-1 and PAI-1 mutants with thrombin

Thrombin, labelled with $^{125}$I employing the Iodogen procedure, was incubated in 20 µl 20 mM Tris-HCl (pH 8.0), 100 mM NaCl, 0.1% (v/v) Tween-80 for 1 h at 37°C with vitronectin, with PAI-1 or with vitronectin and PAI-1. Vitronectin was used in concentrations of 3.5 nM and 70 nM and the inhibitor was used in concentrations of 0.75 nM and 7.5 nM. The amount of labelled thrombin was 2.5 nM in all cases. To the reaction mixtures 5 µl of 0.25 M Tris-HCl (pH 6.8), 10% (w/v) SDS, 40% (v/v) glycerol, 0.05% (w/v) bromophenol blue was added and the final mixtures were analyzed by electrophoresis on a 10% (w/v) SDS-polyacrylamide gel (Laemmli, 1970). Thrombin was visualized by fluorography.

## References

Andreasen et al, FEBS Lett. 209 (1986) 213-218.

Bock et al, Nucleic Acids Res. 10 (1982) 8113-8125.

Carrell and Boswell in: Proteinase Inhibitors (eds. Barrett and Salvesen), Elsevier Science Publishers, Amsterdam, Netherlands, 1986, pp. 403-420.

Chase and Shaw, Meth. Enzymol. 19 (1970) 20-27.

Collen et al, Coronary thrombolysis with recombinant human tissue type plasminogen activator: a prospective, randomized placebo-controlled trial. Circulation 70 (1984) 1012

Declerck et al, Biol. Chem. 263 (1988) 15454-15461.

Esmon, Progress in Haemostasis and Thrombosis 9 (1989) 29-55.

Hekman and Loskutoff, J. Biol. Chem. 260 (1985) 11581-11587.

Hill and Hastie, Nature 326 (1987) 96-99

Keijer et al, Fibrinolysis 2, Suppl. 1 (1988), 119 (abstract).

Kerins et al, Evidence for platelet activation during coronary thrombolysis with tissue plasminogen activator in man. Clin. Res. 36 (1988) A4 (abstract).

Kramer et al, Nucleic Acids Res. 12 (1984) 9441-9456.

Laemmli, Nature 277 (1970) 680-685.

Lambers et al, J. Biol. Chem. 262 (1987) 17492-17496.

Maniatis et al, Molecular Cloning: A Laboratory Manual, Cold Spring Harbor Laboratory Press, Cold Spring Harbor, N.Y., 1982

Mimuro and Loskutoff, J. Biol. Chem. 264 (1989) 936-939. Mimuro et al, Blood 70 (1987) 721-728.

Murayame and Bang, Thromb. Hemost. 58 (1987), 446 (abstract).

Owen et al, Thrombolytic therapy with tissue plasminogen activator or streptokinase induces transient thrombin activity. Blood 72 (1988) 616-620.

Pannekoek et al, EMBO J. 5 (1986) 2539-2544.

Preissner et al, Biochem. J. 231 (1985) 349-355.

Rosenberg and Damus, J. Biol. Chem. 248 (1973) 6490-6505.

Sanger et al, Proc. Natl. Acad. Sci. USA. 74 (1977) 5463-5467.

Schleef and Loskutoff, Haemostasis 18 (1988) 328-341.

TIMI Study Group, 1985. The thrombolysis in myocardial infarction (TIMI) trial. N. Engl. J. Med. 312:932.

Travis and Salvesen, Ann. Rev. Biochem. 52 (1983) 655-709.

Verstraete et al, Acute coronary thrombolysis with recombinant human tissue-type plasminogen activator: Initial patency and influence of maintained infusion on reocclusion rate. Am. J. Cardiol. 60 (1987) 231.

Verstraete et al, Vergleichende randomisierte Untersuchung zur Wirksamkeit von rekombinantem Gewebe-Plasminogen-Aktivator i.v. und Streptokinase i.v. bei Patienten mit akutem Herzinfarkt. Klin. Wochensch. 66 (Suppl. XII, 1988) 77.

Wiman et al, FEBS Lett. 242 (1988) 125-128.

## Claims

1. A mutant of the human plasminogen activator inhibitor (PAI-1) in which (using the one-letter code of amino acids) the amino acids RM contained in the reactive centre region of PAI-1 having the amino acid sequence SGTVASSSTAVIVSARMAPEEIIMD have been replaced by the amino acids RS contained in the reactive centre region of antithrombin III (ATIII) having the amino acid sequence EGSEAAASTAVVIAGRSLNPNRVTFKAN, and optionally flanking amino acids within said PAI-1 reactive centre region have been replaced by the corresponding flanking amino acids within said ATIII reactive centre region.

2. A mutant according to claim 1, in which at least the amino acids RM in the positions P1 and P1' of PAI-1 are replaced by the amino acids RS of ATIII, located in corresponding positions.

3. A mutant according to claim 1, in which at least the amino acids ARMA in the positions P2 through P2' of PAI-1 are replaced by the amino acids GRSL of ATIII, located in corresponding positions.

4. A mutant according to claim 1, in which at least the amino acids SARMAP in the positions P3 through P3' of PAI-1 are replaced by the amino acids AGRSLN of ATIII, located in corresponding positions.

5. A recombinant polynucleotide, characterized in that it codes for a PAI-1 mutant according to any one claims 1-4.

6. A recombinant polynucleotide, comprising a vector part and an insertion part, characterized in that the insertion part comprises a nucleotide sequence which codes for a PAI-1 mutant according to any one of claims 1-4.

7. A method of producing a PAI-1 mutant according to any one of claims 1-4 by culturing an organism which, as a result of genetic manipulation using a recombinant polynucleotide according to claim 6, has acquired the ability to express the PAI-1 mutant and isolating the PAI-1 mutant formed.

8. A pharmaceutical preparation, comprising a PAI-1 mutant according to any one of claims 1-4 and one or more pharmaceutically acceptable carriers and/or adjuvants.

9. Use of a PAI-1 mutant according to any one of claims 1-4 for preparing a pharmaceutical composition for use in a fibrinolytic/thrombolytic therapy with a plasminogen activator for preventing the occurrence of reocclusion.

**Patentansprüche**

1. Mutant des menschlichen Plasminogenaktivator-Inhibitors (PAI-1), wobei (unter Verwendung des Einbuchstabencodes der Aminosäuren) die Aminosäuren RM, enthalten im reaktiven Zentrum des PAI-1 der Aminosäuresequenz SGTVASSSTAVIVSARMAPEEIIMD, durch die Aminosäuren RS, enthalten im reaktiven Zentrum des Antithrombins III (ATIII) der Aminosäuresequenz EGSEAAASTAVVIAGRSLNPNRVTFKAN, ersetzt worden sind und gegebenenfalls flankierende Aminosäuren innerhalb des PAI-1-reaktiven Zentrums durch die entsprechenden flankierenden Aminosäuren innerhalb des ATIII-reaktiven Zentrums ersetzt worden sind.

2. Mutant nach Anspruch 1, wobei mindestens die Aminosäuren RM in den Stellungen P1 und P1' des PAI-1 durch die Aminosäuren RS des ATIII, die sich in entsprechenden Stellungen befinden, ersetzt sind.

3. Mutant nach Anspruch 1, wobei mindestens die Aminosäuren ARMA in den Stellungen P2 bis P2' des PAI-1 durch die Aminosäuren GRSL des ATIII, die sich in entsprechenden Stellungen befinden, ersetzt sind.

4. Mutant nach Anspruch 1, wobei mindestens die Aminosäuren SARMAP in den Stellungen P3 bis P3' des PAI-1 durch die Aminosäuren AGRSLN des ATIII, die sich in entsprechenden Stellungen befinden, ersetzt sind.

5. Rekombinantes Polynucleotid, dadurch gekennzeichnet, daß es für einen PAI-1-Mutanten nach einem der Ansprüche 1 bis 4 codiert.

6. Rekombinantes Polynucleotid, enthaltend einen Vektorteil und einen Insertteil, dadurch gekennzeichnet, daß der Insertteil eine Nukleotidsequenz enthält, die für einen PAI-1-Mutanten nach einem der Ansprüche 1 bis 4 codiert.

7. Verfahren zur Herstellung eines PAI-1-Mutanten nach einem der Ansprüche 1 bis 4 durch Züchtung eines Organismus, der, infolge einer genetischen Manipulation unter Verwendung eines rekombinanten Polynucleotids nach Anspruch 6, die Fähigkeit erworben hat, den PAI-1-Mutanten zur Expression zu bringen und den gebildeten PAI-1-Mutanten zu isolieren.

8. Pharmazeutische Präparation, enthaltend einen PAI-1-Mutanten nach einem der Ansprüche 1 bis 4 und einen oder mehrere pharmazeutisch akzeptable Träger und/oder Hilfsmittel.

9. Verwendung eines PAI-1-Mutanten nach einem der Ansprüche 1 bis 4 zur Herstellung einer pharmazeutischen Zusammensetzung zur Verwendung in einer fibrinolytischen/thrombolytischen Therapie mit einem Plasminogenaktivator zur Verhinderung des Auftretens einer Reokklusion.

**Revendications**

1. Mutant de l'inhibiteur (PAI-1) de l'activateur de plasminogène humain dans lequel (en utilisant le code à une lettre des acides aminés), les acides aminés RM contenus dans la région centrale réactive de PAI-1 possédant la séquence d'acides aminés SGTVASSSTAVIVSARMAPEEIIMD ont été remplacés par les acides aminés RS contenus dans la région centrale réactive de l'antithrombine III (ATIII) possédant la séquence d'acides aminés EGSEAAASTAVVIAGRSLNPNRVTFKAN et, optionnellement, des acides aminés latéraux dans la région centrale réactive PAI-1 ont été remplacés par les acides aminés latéraux correspondants dans ladite région centrale réactive ATIII.

2. Mutant selon la revendication 1, dans lequel au moins les acides aminés RM dans les positions P1 et P1' de PAI-1 ont été remplacés par les acides aminés RS d'ATIII situés dans les positions correspondantes.

3. Mutant selon la revendication 1, dans lequel au moins les acides aminés ARMA dans les positions P2 à P2' de PAI-1 sont remplacés par les acides aminés GRSL d'ATIII situés dans les positions correspondantes.

4. Mutant selon la revendication 1, dans lequel au moins les acides aminés SARMAP dans les positions P3 à P3' de PAI-1 sont remplacés par les acides aminés AGRSLN d'ATIII situés dans les positions correspondantes.

5. Polynucléotide recombinant caractérisé en ce qu'il code le mutant PAI-1 selon l'une quelconque des revendications 1 à 4.

6. Polynucléotide recombinant comprenant une partie de vecteur et une partie d'insertion, caractérisé en ce que la partie d'insertion comprend une séquence de nucléotides qui code le mutant PAI-1 selon l'une quelconque des revendications 1 à 4.

7. Procédé de production d'un mutant PAI-1 selon l'une quelconque des revendications 1 à 4 en cultivant un organisme qui, à la suite d'une manipulation génétique utilisant un polynucléotide recombinant selon la revendication 6, a acquis la capacité d'exprimer le mutant PAI-1 et d'isoler le mutant PAI-1 ainsi formé.

8. Préparation pharmaceutique comprenant un mutant PAI-1 selon l'une quelconque des revendications 1 à 4 et un ou plusieurs excipients et/ou adjuvants pharmaceutiquement compatibles.

9. Utilisation d'un mutant PAI-1 selon l'une quelconque des revendications 1 à 4 pour préparer une composition pharmaceutique à utiliser dans une thérapie fibrinolytique/thrombolytique avec un activateur de plasminogène pour empêcher l'incidence d'une réocclusion.

FIG. 1

FIG. 2